# EUROPÄISCHE PATENTSCHRIFT

(11) **EP 4 590 226 B1**
(45) Veröffentlichungstag und Bekanntmachung des Hinweises auf die Patenterteilung: **23.09.2026**
(21) Anmeldenummer: 23836357.6
(22) Anmeldetag: 15.12.2023
(51) Int. Cl.: A61B 34/00, A61B 46/10, A61B 17/00

(54) **LENKGETRIEBE FÜR EIN CHIRURGISCHES INSTRUMENT**
STEERING MECHANISM FOR A SURGICAL INSTRUMENT
MÉCANISME DE DIRECTION POUR INSTRUMENT CHIRURGICAL

(30) Priorität: 20.12.2022 DE 102022134203
(43) Veröffentlichungstag der Anmeldung: 30.07.2025
(73) Patentinhaber: Karl Storz SE & Co. KG, 78532 Tuttlingen (DE)
(72) Erfinder: GRÜNER, Sven, 78532 Tuttlingen (DE); STEFAN, Jochen, 78532 Tuttlingen (DE)
(86) Internationale Anmeldenummer: PCT/EP2023/086009
(87) Internationale Veröffentlichungsnummer: WO 2024/132904

(56) Entgegenhaltungen:
- CN-A- 103 552 088
- DE-A1- 102019 121 092
- DE-B3- 102021 119 527
- US-A1- 2012 239 060

## Beschreibung

Die vorliegende Erfindung betrifft ein Lenkgetriebe für ein chirurgisches Instrument. Ferner betrifft die vorliegende Erfindung ein chirurgisches Instrument zur Kopplung mit einem solchen Lenkgetriebe. Zudem betrifft die vorliegende Erfindung ein steriles Antriebssystem mit einem Lenkgetriebe und einem chirurgischen Instrument. Die vorliegende Erfindung betrifft auch ein Verfahren zur Montage eines solchen sterilen Antriebssystems.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl von Lenkdrähten oder Lenkseilen verbunden, um eine feinfühlige Steuerung der Werkzeugspitze zu erreichen. Mit den Lenkdrähten kann eine gleichmäßige Kraftverteilung in allen Abwicklungsrichtungen erreicht werden.

CN 103552088 B beschreibt ein Lenkgetriebe für ein chirurgisches Instrument mit einer Taumelscheibe.

Aus US 7,699,855 B2 ist ein chirurgisches Instrument bekannt, das eine Schnittstelle aufweist, um das Instrument mit einem Roboterarm verbinden zu können. Sämtliche Antriebe für die Funktionen des Instruments sind an dem Roboterarm angeordnet. Die Übertragung der Drehwinkel von den Antrieben zum Instrument erfolgt über Kupplungsscheiben in einer gemeinsamen Trennebene. WO 2014/004242 A1 und US 2017/0165017 A1 offenbaren weitere Ausführungen von Schnittstellen zwischen einem chirurgischen Instrument und den roboterseitig angeordneten Antrieben zum Antreiben des chirurgischen Instruments.

Aus DE 10 2019 121 092 A1 ist ein chirurgisches Instrument bekannt, das ein Lenkgetriebe aufweist. Mit dem Lenkgetriebe können die Stellwinkel von zwei Antrieben direkt auf eine räumlich verstellbare Scheibe (Taumelscheibe) übertragen werden, um diese zur Steuerung der Werkzeugspitze auszurichten. Dazu werden an der Taumelscheibe Lenkdrähte befestigt, sodass sich die Werkzeugspitze durch Ausrichtung der Taumelscheibe stufenlos und flüssig steuern lässt. Auch aus US 10,105,128 B2 ist ein Getriebe für ein chirurgisches Instrument bekannt. Das Getriebe dieses chirurgischen Instruments umfasst eine verlagerbare Scheibe und Gelenksstangen, die zum Bewegen der verlagerbaren Scheibe angeordnet sind. Ausgehend von diesem Stand der Technik ist es Aufgabe der vorliegenden Erfindung, ein Lenkgetriebe mit einer Schnittstelle für ein chirurgisches Instrument bereitzustellen, über die eine instrumentenseitig angeordnete Taumelscheibe ansteuerbar ist.

Diese Aufgabe wird mit einem Lenkgetriebe für ein chirurgisches Instrument gemäß dem Patentanspruch 1 gelöst. Weitere Ausführungsformen sind in den abhängigen Ansprüchen angegeben.

Das erfindungsgemäße Lenkgetriebe weist ein Getriebegehäuse und eine Schnittstelle zur Kopplung mit einem chirurgischen Instrument auf. Die Schnittstelle ist derart ausgebildet, dass das chirurgische Instrument in einer Richtung senkrecht zur Längsachse des Lenkgetriebes mit dem Lenkgetriebe koppelbar ist. Die Schnittstelle umfasst einen Gehäusebereich des Getriebegehäuses und eine Lenkklammer zur Betätigung einer Lenkeinrichtung des chirurgischen Instruments. Der Gehäusebereich des Getriebegehäuses ist zur Kopplung mit einem Gehäuse des chirurgischen Instruments ausgebildet. Die Lenkklammer ist mit einer Taumelscheibe der Lenkeinrichtung des chirurgischen Instruments über die Schnittstelle koppelbar.

Das erfindungsgemäße Lenkgetriebe ist zur Ansteuerung einer Lenkeinrichtung eines chirurgischen Instruments über die Schnittstelle ausgebildet. Insbesondere kann über die Schnittstelle des Lenkgetriebes eine Taumelscheibe der Lenkeinrichtung des chirurgischen Instruments gesteuert werden. Das chirurgische Instrument kann von oben auf das Lenkgetriebe aufgesetzt und über die Schnittstelle mit dem Lenkgetriebe gekoppelt werden. Aufgrund der Schnittstelle des Lenkgetriebes kann das chirurgische Instrument relativ einfach und damit kostengünstig aufgebaut werden. Das chirurgische Instrument kann demnach als sogenannter "LimitedUse"-Artikel ausgeführt sein. Die Schnittstelle des Lenkgetriebes ermöglicht einen intraoperativen Wechsel des chirurgischen Instruments. Das sterile Lenkgetriebe bleibt während der Operation durchgängig mit einer roboterseitigen angeordneten Antriebseinheit gekoppelt, während verschiedene chirurgische Instrumente intraoperativ mit dem Lenkgetriebe gekoppelt werden können. Das erfindungsgemäße Lenkgetriebe ist zudem mehrfach sterilisierbar.

Die Lenkklammer kann derart ausgebildet sein, dass die Lenkklammer in einer Richtung senkrecht zur Längsachse mit der Taumelscheibe des chirurgischen Instruments in Eingriff bringbar ist. Das chirurgische Instrument kann dementsprechend in einer Richtung senkrecht zur Längsachse des Lenkgetriebes auf das Lenkgetriebe aufgesetzt werden. Die Lenkklammer ist dementsprechend nach oben geöffnet, sodass die Taumelscheibe von oben mit der Lenkklammer in Eingriff bringbar ist. Dabei kann die Lenkklammer mit der Taumelscheibe gekoppelt werden, sodass die Bewegungen der Lenkklammer auf die instrumentenseitig angeordnete Taumelscheibe übertragen werden können. Auch das Abnehmen des chirurgischen Instruments von dem Lenkgetriebe erfolgt in einer Richtung senkrecht zur Längsachse des Lenkgetriebes. Die Wechsel des chirurgischen Instruments können intraoperativ stattfinden, wobei das Lenkgetriebe während der Operation durchgängig an der Antriebseinheit verbleibt.

Die Lenkklammer kann wenigstens einen elastischen Abschnitt aufweisen. Der elastische Abschnitt kann einen zentralen Bereich der Lenkklammer bilden. Der wenigstens eine elastische Abschnitt kann wellenförmig ausgebildet sein. Die Lenkklammer kann zwei Klammerarme aufweisen. Die beiden Klammerarme können sich ausgehend von dem elastischen Abschnitt nach außen erstrecken. Die beiden Klammerarme können eine Klammeröffnung definieren, in die die Taumelscheibe eingesetzt werden kann, um die Taumelscheibe mit der Lenkklammer zu koppeln.

Die Lenkklammer kann axiale Kontaktflächen für die Taumelscheibe aufweisen. An den axialen Kontaktflächen der Lenkklammer können sich korrespondierende Kontaktflächen der Taumelscheibe abstützen, wenn die Taumelscheibe mit der Lenkklammer gekoppelt ist. Die axialen Kontaktflächen der Lenkklammer sind einander entgegengesetzt an der Lenkklammer ausgebildet. Anders ausgedrückt weisen die axialen Kontaktflächen der Lenkklammer voneinander weg. Die axialen Kontaktflächen der Lenkklammer können sich zumindest im Wesentlichen parallel zueinander erstrecken.

Das Lenkgetriebe kann wenigstens zwei Schieber aufweisen, die jeweils wenigstens einen Kugelkopf haben. Der wenigstens einen Kugelkopf kann mit dem ihm zugeordneten Schieber fest verbunden sein. Die Kugelköpfe an den Schiebern dienen zur Kopplung der Schieber mit der Lenkklammer. Die Kugelköpfe der Schieber sind dabei so ausgebildet, dass die Lenkklammer beweglich an ihnen angebracht werden kann. Veränderungen der beiden Schieber relativ zueinander führen zu Veränderungen der Positionen der Kugelköpfe relativ zueinander, wodurch die Lenkklammer bewegt werden kann.

Die Lenkklammer kann Kugelpfannen aufweisen, mit denen sie mit den Kugelköpfen an den Schiebern verbunden ist. Jeweils eine Kugelpfanne kann an einem der Klammerarme ausgebildet sein. Die Kugelpfannen können an der Außenseite der Klammerarme ausgebildet sein. Der elastische Abschnitt der Lenkklammer ist zwischen den beiden Klammerarmen angeordnet. Der elastische Abschnitt kann somit auch zwischen den beiden Kugelpfannen angeordnet sein. Jeweils ein Kugelkopf an einem der Schieber und eine Kugelpfanne der Lenkklammer können ein Gelenk bilden, das gelenkige Bewegungen oder Auslenkungen der Lenkklammer zulässt.

Bewegungen oder Auslenkungen der Lenkklammer können insbesondere durch einen sich verändernden Abstand der Kugelköpfe relativ zueinander erzeugt werden. Der Abstand der Kugelköpfe kann sich durch Bewegungen der Schieber entlang der Längsachse des Lenkgetriebes verändern, wodurch die Lenkklammer bewegt und auch verkippt werden kann. Der sich verändernde Abstand der Kugelköpfe, die mit den Kugelpfannen der Lenkklammer gekoppelt sind, kann über den elastischen Abschnitt der Lenkklammer kompensiert werden.

Die Schnittstelle kann eine Zugklaue aufweisen, die zur Kopplung mit der Lenkeinrichtung des chirurgischen Instruments ausgebildet ist. Die Zugklaue kann eine Aufnahme aufweisen, die derart ausgebildet ist, dass die Zugklaue in einer Richtung senkrecht zur Längsachse des Lenkgetriebes mit der Lenkeinrichtung des chirurgischen Instruments in Eingriff bringbar ist.

Das Lenkgetriebe kann eine erste Antriebsspindel und eine zweite Antriebsspindel aufweisen. Die Antriebsspindeln können jeweils einen der Schieber antreiben. Die Antriebsspindeln können die Schieber in einer Richtung parallel zur Längsachse des Lenkgetriebes antreiben. Da die beiden Schieber über die ihnen zugeordneten Antriebsspindeln relativ zueinander bewegt werden können, kann sich der Abstand zwischen den Kugelköpfen an den Schiebern verändern. Durch die Veränderung der Relativposition der Schieber zueinander und damit durch den sich verändernden Abstand der Kugelköpfe können Bewegungen der Lenkklammer ausgelöst werden, die auf die Taumelscheibe übertragen werden können.

Die Zugklaue kann über eine dritte Antriebsspindel antreibbar sein. Die Zugklaue kann über die dritte Antriebsspindel entlang der Längsachse des Lenkgetriebes verlagert werden.

Die Antriebsspindeln können sich parallel zur Längsachse des Lenkgetriebes durch das Getriebegehäuse erstrecken und an ihren Enden jeweils eine Antriebsscheibe aufweisen, die mit Motortreibscheiben der roboterseitigen Antriebseinheit koppelbar sind.

Die Schnittstelle kann wenigstens ein Stirnrad zum Antreiben eines Schafts des chirurgischen Instruments aufweisen. Das Stirnrad kann von einer weiteren Antriebsscheibe antreibbar sein. Das Stirnrad kann über ein Getriebe mit der Antriebsscheibe gekoppelt sein. Dieses Getriebe kann von wenigstens zwei Zahnrädern gebildet werden. Das Stirnrad kann über eine Welle mit dem Getriebe verbunden sein. Mit dem Getriebe kann beispielsweise ein Versatz in einer Richtung senkrecht zur Längsachse des Lenkgetriebes zwischen dem Stirnrad und der Antriebsscheibe ausgeglichen werden. Das wenigstens eine Stirnrad kann in Richtung der Längsachse des Getriebegehäuses zwischen der Lenkklammer und der Zugklaue angeordnet sein.

Alternativ kann eine Welle mit einem Zahnrad verbunden sein, das drehmomentübertragend mit dem Stirnrad zum Antreiben des Schafts des chirurgischen Instruments gekoppelt ist. Das Stirnrad kann an der der Zugklaue abgewandten Seite der Lenkklammer angeordnet sein. Das Stirnrad kann an einem den Antriebsscheiben abgewandten Ende des Getriebegehäuses des Lenkgetriebes angeordnet sein.

Das Lenkgetriebe kann eine weitere Schnittstelle zur Verbindung mit einer Antriebseinheit aufweisen. Diese Schnittstelle kann die Antriebsscheiben zur Kopplung mit den Motortreibscheiben einer Antriebseinheit aufweisen. Das Lenkgetriebe kann somit eine erste Schnittstelle zur Kopplung mit dem chirurgischen Instrument und eine zweite Schnittstelle zur Kopplung mit der Antriebseinheit aufweisen. Diese Antriebseinheit kann roboterseitigen angeordnet sein. Die Kopplung mit der Antriebseinheit kann in Richtung der Längsachse des Lenkgetriebes erfolgen. Die Richtung der Längsachse kann der Richtung der Drehachsen der Antriebsspindeln des Lenkgetriebes entsprechen, die parallel zur Längsachse des Lenkgetriebes verlaufen.

Mit der zweiten Schnittstelle kann das Lenkgetriebe die unsterilen Antriebe an der Antriebseinheit abdecken, sodass das Lenkgetriebe während einer Operation durchgängig mit der Antriebseinheit bzw. den darin enthaltenen Antrieben gekoppelt bleiben kann. An der zweiten Schnittstelle des Lenkgetriebes können die Antriebsscheiben der Antriebsspindeln angeordnet sein. Insbesondere kann die zweite Schnittstelle eine sich im Wesentlichen senkrecht zur Längsachse des Lenkgetriebes verlaufende Fläche des Getriebegehäuses umfassen, an der die Antriebsscheiben angeordnet sind. An der Antriebseinheit können die Motortreibscheiben angeordnet sein, die drehmomentübertragend mit den Antriebsscheiben gekoppelt werden können, um die Antriebsspindeln des Lenkgetriebes anzutreiben.

Die vorliegende Erfindung betrifft ferner ein chirurgisches Instrument zur Kopplung mit einer Schnittstelle eines Lenkgetriebes gemäß der voranstehend beschriebenen Art. Das chirurgische Instrument umfasst ein Gehäuse und eine in dem Gehäuse angeordnete Lenkeinrichtung. Die Lenkeinrichtung kann zumindest eine Schaftwelle, eine Taumelscheibe und mit der Taumelscheibe verbundene Lenkdrähte aufweisen. Das Gehäuse hat eine Öffnung, über die die Lenkeinrichtung mit dem Lenkgetriebe koppelbar ist.

Das chirurgische Instrument ist so ausgebildet, dass es in einer Richtung senkrecht zur Längsachse des chirurgischen Instruments mit der Schnittstelle des Lenkgetriebes koppelbar ist. Aufgrund des Lenkgetriebes bzw. der an dem Lenkgetriebe ausgebildeten Schnittstelle kann das chirurgische Instrument den voranstehend beschriebenen einfachen und damit kostengünstigen Aufbau aufweisen. Das chirurgische Instrument selbst kann aufgrund seines einfachen Aufbaus ein sogenannter Limited-use-Artikel sein.

Die Taumelscheibe kann eine umlaufende Nut aufweisen, die axiale Kontaktflächen für die Lenkklammer hat. Die umlaufende Nut der Taumelscheibe kann mit der Lenkklammer in Eingriff gebracht werden. Die Lenkklammer kann in der Nut zumindest abschnittsweise aufgenommen werden. Durch die korrespondierenden axialen Kontaktflächen an der Nut der Taumelscheibe und den axialen Kontaktflächen an der Lenkklammer kann ein Verkanten der Taumelscheibe bei der Übertragung von Bewegungen von der Lenkklammer auf die Taumelscheibe des chirurgischen Instruments verhindert werden.

Die Taumelscheibe kann Befestigungselemente zur Befestigung der Lenkdrähte an der Taumelscheibe aufweisen. Dazu können an der Taumelscheibe Befestigungsöffnungen ausgebildet sein, die sich in radialer Richtung in die Taumelscheibe hinein erstrecken. Die Befestigungselemente können in den Befestigungsöffnungen aufgenommen sein und eine Klemmkraft für die an der Taumelscheibe zu befestigenden Lenkdrähte bereitstellen. Die Befestigungselemente können Schrauben sein. Dementsprechend können die Befestigungsöffnungen in der Taumelscheibe ein Innengewinde aufweisen. Insbesondere können die Befestigungselemente Madenschrauben sein.

Die Lenkeinrichtung kann wenigstens eine Zughülse und ein daran angeordnetes Wälzlager umfassen. Die Zughülse und das daran angeordnete Wälzlager können zum Einsetzen in die Aufnahme der Zugklaue des Lenkgetriebes ausgebildet sein. Die Zughülse kann im mit der Zugklaue gekoppelten Zustand die Bewegungen der Zugklaue entlang der Längsachse des chirurgischen Instruments auf eine Zugstange des chirurgischen Instruments übertragen.

Die Lenkeinrichtung kann wenigstens ein Zahnrad aufweisen, das an der Schaftwelle angeordnet und mit dem Stirnrad des Lenkgetriebes in Eingriff bringbar ist. Mit dem an der Schaftwelle angeordneten Zahnrad kann die Schaftwelle über das Stirnrad des Lenkgetriebes drehangetrieben werden, um die Schaftwelle in eine Rotation zu versetzen. Das wenigstens eine Zahnrad kann in Längsrichtung zwischen der Taumelscheibe und der Zughülse an der Schaftwelle angeordnet sein. Ferner kann das wenigstens eine Zahnrad in Längsrichtung zwischen der Taumelscheibe und einer Wand des Gehäuses des chirurgischen Instruments angeordnet sein, die sich senkrecht zur Längsachse erstreckt.

Die vorliegende Erfindung betrifft ferner ein steriles Antriebssystem für ein chirurgisches Instrument der voranstehend beschriebenen Art. Das Antriebssystem weist ein Lenkgetriebe und eine Antriebseinheit auf, die an einem Roboterarm anbringbar ist. Das Antriebssystem umfasst ferner wenigstens einen sterilen Überzug, der zumindest die Antriebseinheit umgibt, wobei der sterile Überzug eine Schnittstelle der Antriebseinheit freigibt. Das sterile Lenkgetriebe ist direkt mit der Schnittstelle an der Antriebseinheit koppelbar, wobei die Schnittstelle durch das Lenkgetriebe abgedeckt wird. Das sterile chirurgische Instrument kann mit der Schnittstelle an dem Lenkgetriebe austauschbar gekoppelt werden.

Wie voranstehend beschrieben wurde, weist das Lenkgetriebe neben der ersten Schnittstelle für das chirurgische Instrument auch eine zweite Schnittstelle zur Kopplung mit der Schnittstelle der Antriebseinheit auf, die an einem Roboterarm anbringbar ist. Das Lenkgetriebe kann direkt mit der Schnittstelle der Antriebseinheit gekoppelt werden. Dies bedeutet, dass die Kopplung ohne zwischengeschaltete Elemente wie zum Beispiel einem Steriladapter erfolgen kann. Mit anderen Worten deckt das Lenkgetriebe die unsterile Schnittstelle an der Antriebseinheit unmittelbar ab. Das Lenkgetriebe kann während einer Operation durchgängig mit der unsterilen Schnittstelle der Antriebseinheit gekoppelt bleiben und diese abdecken.

Die Antriebseinheit kann beispielsweise über eine Führungseinrichtung an dem Roboterarm angebracht sein. Die Führungseinrichtung kann in Form einer Führungsschiene ausgebildet sein.

Der sterile Überzug kann beispielsweise von einer Folie gebildet werden. Der sterile Überzug kann ein Fenster aufweisen, das die Schnittstelle an der Antriebseinheit freigibt, sodass diese direkt mit dem Lenkgetriebe gekoppelt und von diesem abgedeckt werden kann. Durch diese Abdeckung der unsterilen Schnittstelle der Antriebseinheit mit dem Lenkgetriebe kann der sterile Überzug relativ einfach gestaltet sein. Es ist dementsprechend nicht nötig einen Steriladapter in den sterilen Überzug zu integrieren oder einen Steriladapter mit dem sterilen Überzug zu verbinden.

Zudem betrifft die vorliegende Erfindung ein Verfahren zur Montage eines sterilen Antriebssystems für ein voranstehend beschriebenes chirurgisches Instrument, wobei das Antriebssystem ein steriles Lenkgetriebe und eine an einem Roboterarm anbringbare Antriebseinheit aufweist. Das Verfahren umfasst die folgenden Schritte:
Anbringen eines sterilen Überzugs zumindest an der Antriebseinheit, wobei der sterile Überzug eine Schnittstelle der Antriebseinheit freigibt,
Anbringen des sterilen Lenkgetriebes an der Schnittstelle der Antriebseinheit, wobei das sterile Lenkgetriebe direkt mit der Schnittstelle gekoppelt wird, und wobei das sterile Lenkgetriebe die Schnittstelle der Antriebseinheit abdeckt, und
Koppeln eines sterilen chirurgischen Instruments mit der Schnittstelle an dem Lenkgetriebe, wobei das chirurgische Instrument austauschbar ist.

Das sterile Lenkgetriebe kann direkt und damit ohne zwischengeschaltete Elemente an der Schnittstelle der Antriebseinheit angebracht und mit dieser gekoppelt werden. Es ist kein Steriladapter für die Schnittstelle an der Antriebseinheit nötig, da diese Schnittstelle von dem Lenkgetriebe vollständig abgedeckt wird. Es liegt somit eine unmittelbare Kopplung zwischen dem Lenkgetriebe und der Schnittstelle der Antriebseinheit vor.

Im Folgenden wird die Erfindung anhand von Figuren beispielhaft erläutert. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.#Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Es stellen dar:
- Figur 1: eine perspektivische Ansicht eines Lenkgetriebes gemäß einer ersten Ausführungsform der Erfindung;
- Figur 2: eine perspektivische Ansicht eines chirurgischen Instruments gemäß einer ersten Ausführungsform der Erfindung;
- Figur 3: eine perspektivische Ansicht eines Lenkgetriebes gemäß einer zweiten Ausführungsform der Erfindung im mit einem chirurgischen Instrument gemäß einer zweiten Ausführungsform gekoppelten Zustand;
- Figur 4: eine perspektivische Ansicht einer an einem Roboterarm anbringbaren Antriebseinheit mit einem sterilen Überzug;
- Figur 5: eine perspektivische Ansicht der Antriebseinheit gemäß Figur 4 im mit dem Lenkgetriebe gekoppelten Zustand; und
- Figur 6: eine perspektivische Ansicht der Antriebseinheit, des Lenkgetriebes und des chirurgischen Instruments im gekoppelten Zustand.

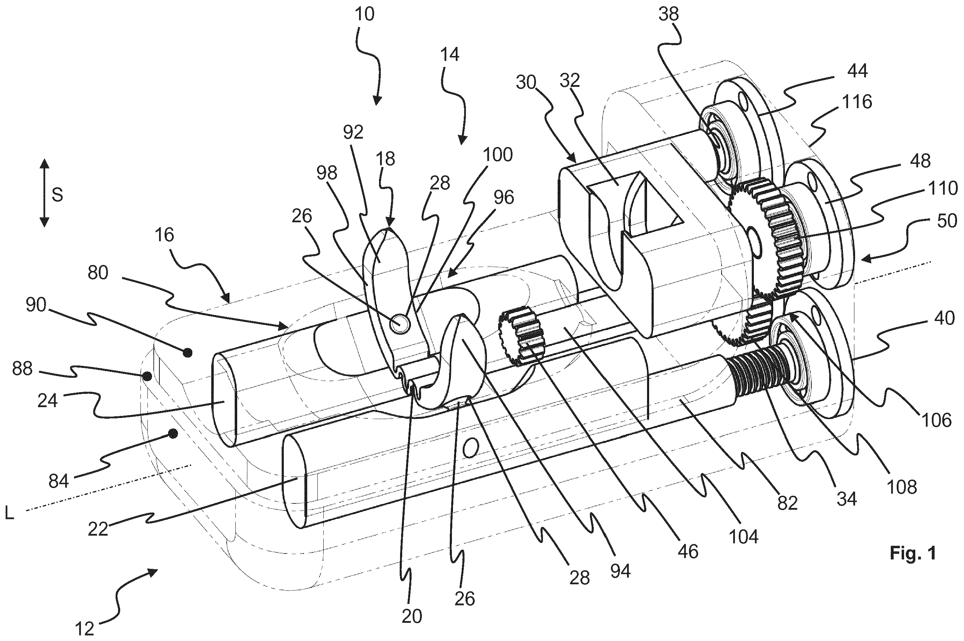

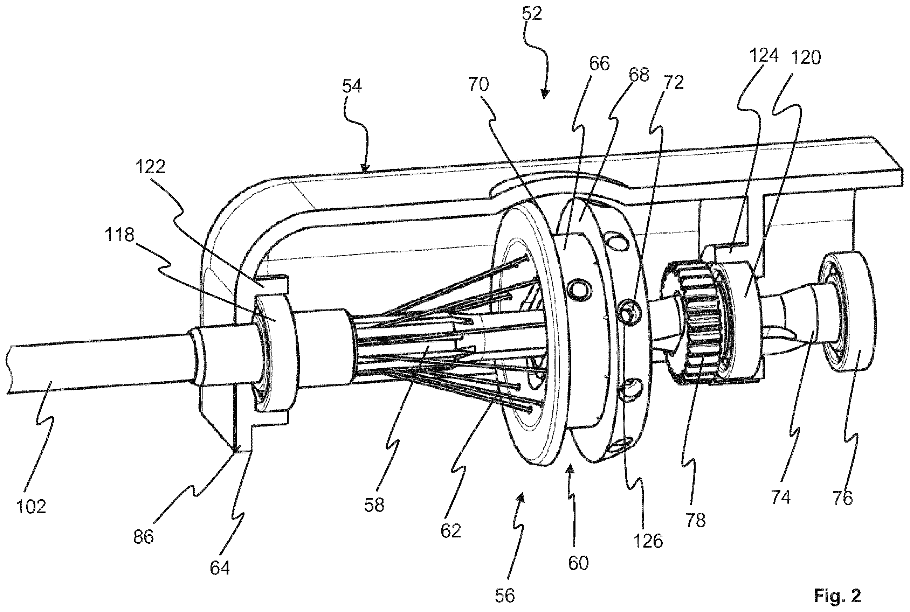

Figur 1 und 2 zeigt eine perspektivische Ansicht eines Lenkgetriebes, das allgemein mit 10 bezeichnet ist. Das Lenkgetriebe 10 weist ein Getriebegehäuse 12 und eine Schnittstelle 14 auf, die zur Kopplung mit einem chirurgischen Instrument 52 ausgebildet ist. Die Schnittstelle 14 umfasst einen Gehäusebereich 16 des Getriebegehäuses 12, der zur Kopplung mit einem Gehäuse 54 des chirurgischen Instruments 52 (siehe Figur 2) ausgebildet ist. Der Gehäusebereich 16 weist einen Aufnahmebereich 80 und eine Aussparung 82 auf. Die Aussparung 82 erstreckt sich an der Außenseite 84 des Gehäuses 12 entlang des Gehäusebereichs 16. Die Aussparung 82 ist derart ausgebildet, dass ein Rand 86 des Gehäuses 54 des chirurgischen Instruments 52 bündig mit der Außenseite 84 des Getriebegehäuses 12 abschließen kann. Die Aussparung 82 bildet eine Anlagefläche 88 für den Rand 86 des Gehäuses 72 aus (siehe Figur 2).

Der Aufnahmebereich 80 erstreckt sich von einer Oberseite 90 des Gehäusebereichs 16 in das Getriebegehäuse 12 hinein. Der Aufnahmebereich 78 dient zur Aufnahme verschiedener Komponenten und Elemente des Lenkgetriebes 10, die nachstehend erläutert werden.

Die Schnittstelle 14 umfasst eine Lenkklammer 18 zur Betätigung einer Lenkeinrichtung 56 des chirurgischen Instruments 52. Die Lenkklammer 18 ist in der Richtung S mit der Taumelscheibe 60 des chirurgischen Instruments 52 in Eingriff bringbar. Die Lenkklammer 18 weist einen elastischen Abschnitt 20 auf. Der elastische Abschnitt 20 ist in der gezeigten Ausführungsform an seiner Wellenform erkennbar. Neben dem elastischen Abschnitt 20 weist die Lenkklammer 18 zwei Klammerarme 92 und 94 auf. Der wellenförmige elastische Abschnitt 20 verbindet die Klammerarme 92 und 94 miteinander. Die Klammerarme 92 und 94 legen zwischen sich eine Klammeröffnung 96 fest, über die die Taumelscheibe 60 mit der Lenkklammer 18 in Eingriff bringbar ist. Dementsprechend ist die Lenkklammer 18 in Richtung S, d. h. nach oben geöffnet. Die Taumelscheibe 60 kann in Richtung S, d.h. von oben, in die Lenkklammer 18 eingesetzt werden.

Die Lenkklammer 18 weist ferner axiale Kontaktflächen 98 und 100 für die Taumelscheibe 60 auf. An den axialen Kontaktflächen 98 und 100 der Lenkklammer 18 kann sich die Taumelscheibe 60 abstützen. Die axialen Kontaktflächen 98 und 100 weisen voneinander weg, d. h. sind einander entgegengesetzt an der Lenkklammer 18 ausgebildet. Die Kontaktflächen 98 und 100 erstrecken sich im Wesentlichen parallel zueinander.

Das Lenkgetriebe 10 weist Schieber 22 und 24 auf, die parallel zur Längsachse L des Lenkgetriebes 10 verlagerbar sind. Die Schieber 22 und 24 können die Lenkklammer 18 bewegen. Dazu sind die Schieber 22 und 24 mit der Lenkklammer 18 gekoppelt. Jeder der beiden Schieber 22 und 24 weist einen Kugelkopf 26 auf, der fest an dem jeweiligen Schieber 22, 24 angebracht ist. Über die Kugelköpfe 26 sind die Schieber 22, 24 mit der Lenkklammer 18 gekoppelt. Die Lenkklammer 18 weist Kugelpfannen 28 auf, in denen jeweils einer der Kugelköpfe 26 aufgenommen ist. Die Lenkklammer 18 ist über die Kugelköpfe 26 beweglich mit den Schiebern 22 und 24 verbunden. Jeweils ein Kugelkopf 26 bildet mit einer Kugelpfanne 28 der Lenkklammer 18 ein Gelenk, sodass die Lenkklammer 18 gelenkige Bewegungen ausführen kann.

Die Kugelpfannen 28 sind in den Klammerarmen 92 und 94 ausgebildet. Zwischen den Kugelpfannen 28 erstreckt sich der elastische Abschnitt 20 der Lenkklammer 18. Durch axiale Relativbewegungen, d.h. Bewegungen entlang der Längsachse L, der Schieber 22 und 24 relativ zueinander, d. h. durch Veränderungen der Relativpositionen der Schieber 22 und 24 in axialer Richtung relativ zueinander, kann die Lenkklammer 18 bewegt werden. Durch die Bewegungen der Schieber 22 und 24 relativ zueinander kann sich der Abstand zwischen den Kugelköpfen 26 der beiden Schieber 22 und 24 und damit der Abstand zwischen den Kugelpfannen 28 der Klammerarme 92 und 94 verändern. Diese Veränderungen des Abstands zwischen den Kugelköpfen 26 und damit zwischen den Kugelpfannen 28 der Lenkklammer 28 können über den elastischen Abschnitt 20 der Lenkklammer 18 kompensiert werden. Die durch die Bewegungen der Schieber 22 und 24 erzeugten Bewegungsmuster der Lenkklammer 18 können auf die Taumelscheibe 60 übertragen werden, wenn das chirurgische Instrument 52 mit dem Lenkgetriebe 10 gekoppelt ist.

Die Schnittstelle 14 weist ferner eine Zugklaue 30 auf. Die Zugklaue 32 kann mit der Lenkeinrichtung 56 des chirurgischen Instruments 52 gekoppelt werden. Die Zugklaue 30 hat eine Aufnahme 32, die in der Richtung S, d.h. nach oben geöffnet ist. Dadurch kann die Zugklaue 30 in Richtung S mit der Lenkeinrichtung 56 des chirurgischen Instruments 52 in Eingriff gebracht werden.

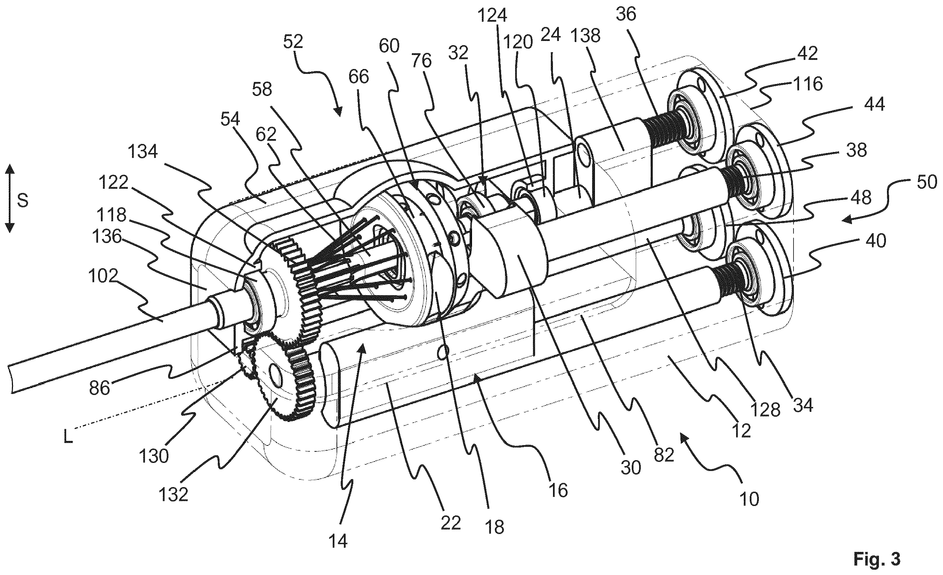

Das Lenkgetriebe 10 weist ein erste Antriebsspindel 34 und eine zweite Antriebsspindel 36 auf, wobei die zweite Antriebspindel in Figur 1 nicht gezeigt ist. In Figur 3 ist die zweite Antriebsspindel 36 dargestellt. Die erste Antriebsspindel 34 treibt den ersten Schieber 22 und die zweite Spindel 36 den zweiten Schieber 24 an. Beide Antriebsspindeln 34 und 36 sind jeweils mit einer Antriebsscheibe 40, 42 verbunden. Die an der zweiten Antriebsspindel 36 angeordnete Antriebsscheibe 42 ist in Figur 3 gezeigt. Die Zugklaue 30 ist mit einer dritten Antriebsspindel 38 verbunden, die die Zugklaue 30 entlang der Längsachse L verlagern kann. Die Antriebsspindeln 34, 36 und 38 erstrecken sich parallel zur Längsachse L des Lenkgetriebes 10 durch das Getriebegehäuse 12.

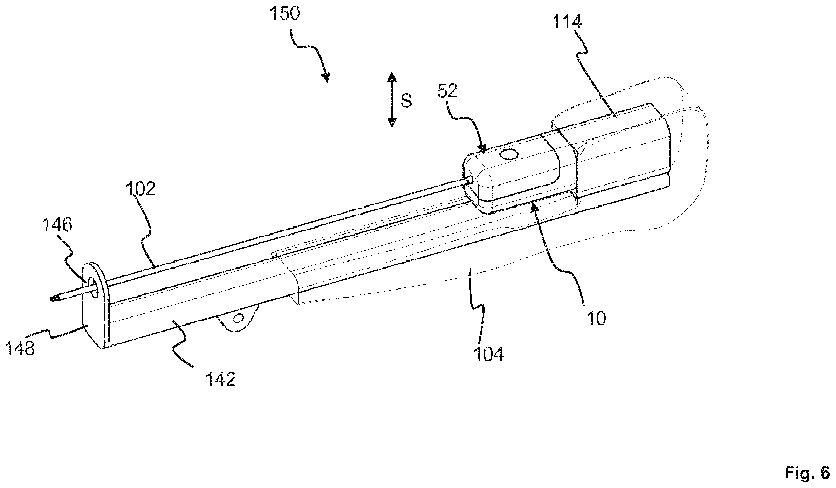

Das Lenkgetriebe 10 weist ein Stirnrad 46 zum Antreiben eines Schafts 102 (siehe Figuren 2 und 6) des chirurgischen Instruments 52 auf. Das Stirnrad ist mit einem Zahnrad 78 gekoppelt, das an einer Schaftwelle 58 des chirurgischen Instruments 52 (siehe Figur 2) angeordnet ist. Das Stirnrad 46 ist über die Welle 104 und ein Zahnradgetriebe 106, das die Zahnräder 108 und 110 umfasst, mit der Antriebsscheibe 48 verbunden. Durch das Zahnradgetriebe 106 kann ein Versatz in der Richtung S zwischen dem Stirnrad 46 und der Antriebsscheibe 48 ausgeglichen werden.

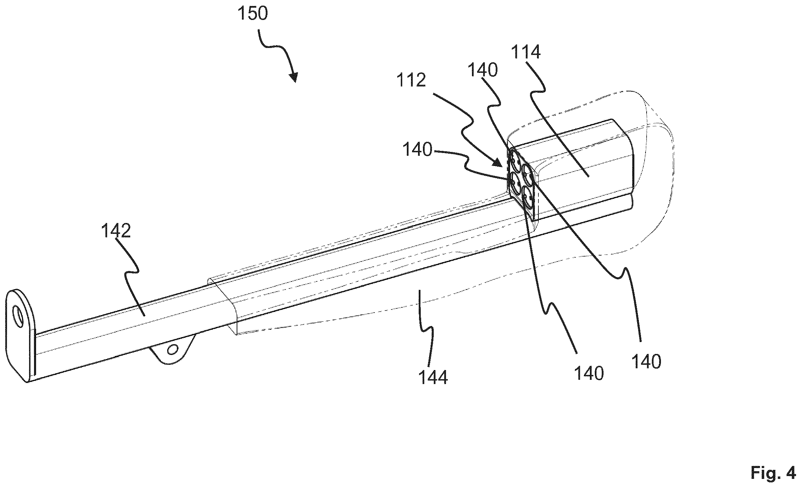

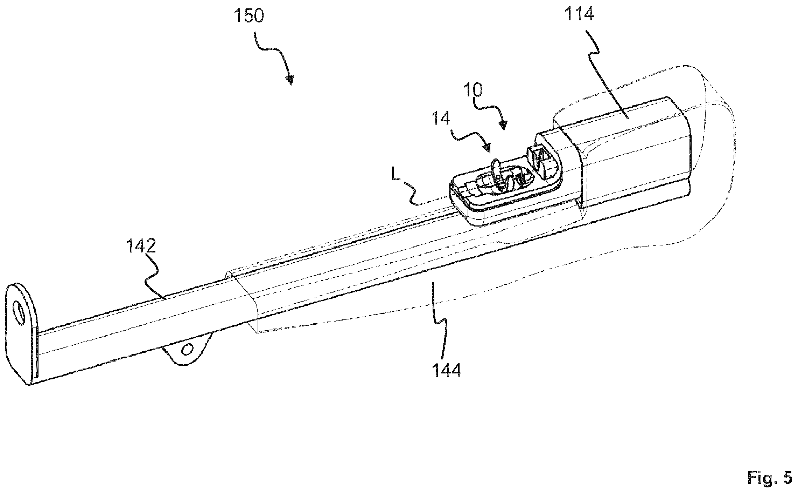

Das Lenkgetriebe 10 weist ferner eine (zweite) Schnittstelle 50 auf. Die zweite Schnittstelle 50 des Lenkgetriebes 10 ist zur Kopplung mit einer Schnittstelle 112 einer Antriebseinheit 114 ausgebildet, die an einem Roboterarm anbringbar ist (siehe Figur 4). Die zweite Schnittstelle 50 umfasst die voranstehend beschriebenen Antriebsscheiben 40, 42, 44, 48. Die Antriebsscheiben 40, 42, 44, 48 sind mit korrespondierenden Motortreibscheiben an der Schnittstelle 112 der Antriebseinheit 114 drehmomentübertragend koppelbar. Die zweite Schnittstelle 50 an dem Lenkgetriebe 10 ist derart ausgebildet, dass die Schnittstelle 112 an der Antriebseinheit 114 von dem Lenkgetriebe 10 verdeckt werden kann (siehe Figur 5).

Das Lenkgetriebe 10 weist somit nicht nur die erste Schnittstelle 14 sondern auch eine zweite Schnittstelle 50 auf. Die zweite Schnittstelle 50 kann an einem in Richtung der Längsachse L des Lenkgetriebes 10 von der ersten Schnittstelle 14 abgewandten Ende des Getriebegehäuses 12 ausgebildet. Die Schnittstelle 50 kann an einer sich senkrecht zur Längsachse L erstreckenden Kopplungsfläche 116 des Getriebegehäuses 12 ausgebildet sein. Die Antriebsscheiben 40, 42, 44, 48 können quer und/oder in der Richtung S senkrecht zur Längsachse L versetzt zueinander an der Kopplungsfläche 116 angeordnet sein.

Die Kopplung zwischen dem Lenkgetriebe 10 und der Antriebseinheit 114 erfolgt in Richtung der Längsachse L. Die Kopplung erfolgt demensprechend auch in Richtung der Drehachsen der Antriebsspindeln 34, 36, 38, da sich diese parallel zur Längsachse L erstrecken. Das Zahnradgetriebe106 kann in Längsrichtung L zwischen der Zugklaue 30 und der zweite Schnittstelle 50 mit den Antriebsscheiben 40, 42, 44, 48 angeordnet sein.

Figur 2 zeigt eine perspektivische Darstellung des chirurgischen Instruments 52. Das chirurgische Instrument 52 weist das Gehäuse 54 auf. In dem Gehäuse 54 ist eine Lenkeinrichtung 56 angeordnet. Die Lenkeinrichtung 56 weist eine Schaftwelle 58, die Taumelscheibe 60 und die mit der Taumelscheibe 60 verbundenen Lenkdrähte 62 auf. An der Schaftwelle 58 sind eine Zughülse 74, ein Wälzlager 76 und das Zahnrad 78 angeordnet, das dazu dient, den Schaft 102 in eine Rotation zu versetzen. Die Zughülse 74 und das daran angeordnete Wälzlager 74 können in die Aufnahme 32 der Zugklaue 30 des Lenkgetriebes 10 eingesetzt werden.

Die Lenkeinrichtung 56 ist über Wälzlager 118 und 120 drehbar an dem Gehäuse 54 gelagert. Dazu sind entsprechende Lagerstellen 122 und 124 an dem Gehäuse 54 ausgebildet. Das Gehäuse 54 hat ferner die Öffnung 64, über die die Lenkeinrichtung 56 mit der Schnittstelle 14 des Lenkgetriebes 10 koppelbar ist. Die Öffnung 64 wird zumindest teilweise durch den Rand 86 des Gehäuses 54 begrenzt. Der Rand 86 gelangt mit der Aussparung 86 an dem Getriebegehäuse 12 in Kontakt. Die Stirnfläche des Rands 86 kann sich dabei an die Anlagefläche 88 an dem Getriebegehäuse 12 anlegen.

Die Taumelscheibe 60 weist eine umlaufende Nut 66 auf. Die umlaufende Nut 66 hat axiale Kontaktflächen 68 und 70 für die Lenkklammer 18. Die axialen Kontaktflächen 68 und 70 sind einander zugewandt. Die axialen Kontaktflächen 68 und 70 können sich parallel zueinander erstrecken. Die korrespondierenden Kontaktflächen 98 und 100 der Lenkklammer 18 sind zum Kontaktieren bzw. zur Anlage an den Kontaktflächen 68 und 70 der Nut 66 ausgebildet. Wenn die Taumelscheibe 60 mit der Lenkklammer 18 gekoppelt ist, wird die Lenkklammer 18 zumindest abschnittsweise in der Nut 66 der Taumelscheibe 60 aufgenommen. Die axialen Kontaktflächen 68, 70 der Nut 66 und die axialen Kontaktflächen 98, 100 der Lenkklammer 18 stehen in diesem Fall miteinander in Kontakt und können sich aneinander abstützen. Durch diesen axialen flächigen Kontakt zwischen den Kontaktflächen 68, 70, 98 und 100 wird bei der Übertragung von Schwenk- oder Kippbewegungen von der Lenkklammer 18 auf die Taumelscheibe 60 ein Verkanten der Taumelscheibe 60 im mit der Lenkklammer 18 gekoppelten Zustand verhindert.

Die Taumelscheibe 60 weist Befestigungselemente 72 zur Befestigung der Lenkdrähte 62 an der Taumelscheibe 60 auf. Dazu sind in Richtung der Längsachse L neben der umlaufenden Nut 66 sich in radialer Richtung erstreckende Befestigungsöffnungen 126 in der Taumelscheibe 60 ausgebildet. In den Befestigungsöffnungen 126 sind die Befestigungselemente 72 aufgenommen. Die Befestigungselemente 72 können Schrauben und insbesondere Madenschrauben sein.

Figur 3 zeigt eine perspektivische Ansicht, die ein Lenkgetriebe 10 gemäß einer zweiten Ausführungsform zeigt. Zudem ist in Figur 3 ein chirurgisches Instrument 52 gemäß einer zweiten Ausführungsform gezeigt. Das Lenkgetriebe 10 und das chirurgische Instrument 52 sind im miteinander gekoppelten Zustand dargestellt.

Der Aufbau des Lenkgetriebes 10 gemäß Figur 3 entspricht weitgehend dem Aufbau des Lenkgetriebes 10 gemäß Figur 1. Die folgende Beschreibung fokussiert sich dementsprechend auf die Unterschiede zwischen diesen beiden Ausführungsformen.

Das Lenkgetriebe 10 gemäß der zweiten Ausführungsform weist das Getriebegehäuse 12 und die Schnittstelle 14 auf, die zur Kopplung mit einem chirurgischen Instrument 52 ausgebildet ist. Die Schnittstelle 14 umfasst einen Gehäusebereich 16 des Getriebegehäuses 12, der zur Kopplung mit einem Gehäuse 54 des chirurgischen Instruments 52 ausgebildet ist. Der Gehäusebereich 16 hat eine Aussparung 82. Die Aussparung 82 erstreckt sich an der Außenseite 84 des Gehäuses 12 entlang des Gehäusebereichs 16. Die Aussparung 82 ist derart ausgebildet, dass der Rand 86 des Gehäuses 54 des chirurgischen Instruments 52 bündig mit der Außenseite 84 des Getriebegehäuses 12 abschließt. Die Schnittstelle 14 weist die Zugklaue 30 mit der nach oben geöffneten Aufnahme 32 auf.

Das Lenkgetriebe 10 umfasst die erste Antriebsspindel 34 und eine zweite Antriebsspindel 36 auf, wobei in Figur 3 auch die zweite Antriebsspindel 36 gezeigt ist. Die erste Antriebsspindel 34 ist dem ersten Schieber 22 zugeordnet. Die zweite Antriebsspindel 36 ist dem zweiten Schieber 24 zugeordnet. Die dritte Antriebsspindel 38 treibt die Zugklaue 30 an, um diese entlang der Längsachse L verlagern zu können.

Die Antriebsspindeln 34, 36, 38 sind mit den Antriebsscheiben 40, 42 und 44 verbunden. Eine weitere Antriebsscheibe 48 ist über eine Welle 128 und ein Zahnrad 130 mit einem Stirnrad 132 drehmomentübertragend gekoppelt. Das Stirnrad 132 dient zum Antreiben des Zahnrades 134 an der Schaftwelle 58 des chirurgischen Instruments 52. Das Zahnrad 134 treibt den Schaft 102 an. Das Stirnrad 132 gemäß dieser Ausführungsform erfüllt somit den gleichen Zweck wie das Stirnrad 46 gemäß der in Figur 1 gezeigte Ausführungsform. Das Stirnrad 132 ist jedoch entlang der Längsachse L an der der Zugklaue 30 abgewandten Seite der Lenkklammer 18 vorgesehen.

Auch das Zahnrad 134 des chirurgischen Instruments 52 ist anders als bei dem chirurgischen Instrument 52 gemäß Figur 2 näher an dem Schaft 102 angeordnet. Das Zahnrad 134 ist gemäß der zweiten Ausführungsform zwischen den Lenkdrähten 62 und einem Wandabschnitt 136 des Gehäuses 54 angeordnet. Der Wandabschnitt 136 erstreckt sich zumindest abschnittsweise senkrecht zur Längsachse L oder zur Schaftachse des Schafts 102 des chirurgischen Instruments 52. An diesem Wandabschnitt 136 ist die Lagerstelle 122 für das Wälzlager 118 ausgebildet. Der Wandabschnitt 136 legt in Richtung der Längsachse L ein Ende des Gehäuses 54 fest. Mit anderen Worten ist das Zahnrad 134 an einem mit Bezug auf den Schaft 102 vorderen Ende des Gehäuses 54 des chirurgischen Instruments 52 angeordnet. Zudem ist das Wälzlager 76, das an der Schaftwelle 58 angeordnet ist, bei der in Figur 3 gezeigten Ausführungsform des chirurgischen Instruments 52 zwischen der Taumelscheibe 60 und dem Wälzlager 120 angeordnet, über das die Schaftwelle 58 an dem Gehäuse 54 gelagert ist. Bei der in Figur 2 gezeigten Ausführungsform ist das Wälzlager 120 zur Lagerung der Schaftwelle 58 an dem Gehäuse 54 zwischen der Taumelscheibe 60 und dem Wälzlager 76 an der Zughülse 74 angeordnet.

Die Antriebsscheiben 40, 42, 44 und 48 sind an der zweiten Schnittstelle 50 des Lenkgetriebes 10 angeordnet. Insbesondere befinden sich die Antriebsscheiben 40, 42, 44, 48 an der Kopplungsfläche 116 des Getriebegehäuses 12, die sich senkrecht zur Längsachse L erstreckt. Die der ersten Antriebsspindel 34 zugeordnete Antriebsscheibe 40 und die der zweiten Antriebsspindel 36 zugeordnete Antriebsscheibe 42 sind in einer Richtung quer zur Längsachse L und in Richtung S zueinander versetzt an der Kopplungsfläche 116 angeordnet. Anders ausgedrückt sind die Antriebsscheiben 40 und 42 schräg zueinander versetzt an der Kopplungsfläche 116 der zweiten Schnittstelle 50 angeordnet. Die zweite Antriebsspindel 36 ist aus diesem Grund über ein Verbindungsstück 138 mit dem zweiten Schieber 24 verbunden. Mit dem Verbindungsstück 138 wird insbesondere der Versatz in Richtung S zwischen der zweiten Antriebsspindel 36 und dem ihr zugeordneten zweiten Schieber 24 ausgeglichen. Ferner wird auch der Versatz in Richtung S zwischen den Antriebswellen 34, 36 über das Verbindungsstück 138 ausgeglichen.

In dem in Figur 3 gezeigten gekoppelten Zustand stützt sich der Rand 86 des Gehäuses 54 des chirurgischen Instruments 52 an der Aussparung 82 ab. Das Gehäuse 54 schließt dementsprechend bündig mit dem Getriebegehäuse 12 ab. Die Lenkklammer 18 ist in der Nut 66 der Taumelscheibe 60 aufgenommen. Das Wälzlager 76 ist in der Aufnahme 32 der Zugklaue 30 aufgenommen. Im gekoppelten Zustand befindet sich die Zugklaue 30 gemäß der in Figur 3 gezeigten Ausführungsform zwischen der Taumelscheibe 60 und dem Wälzlager 120.

Die über die Schieber 22, 24 und die Kugelköpfe 26 (siehe Figur 1) erzeugten Bewegungsmuster der Lenkklammer 18 werden über die Lenkklammer 18 auf die Taumelscheibe 60 übertragen. Da die Kontaktflächen 98 und 100 der Lenkklammer 18 und die Kontaktflächen 68 und 70 der Taumelscheibe 60 flächig aneinander anliegen, kann ein Verkanten der Taumelscheibe 60 verhindert und eine sichere Übertragung der Bewegungen auf die Taumelscheibe 60 erreicht werden. Durch die Bewegungen der Taumelscheibe 60 werden die Lenkdrähte 62 angesteuert, die wiederum die Schwenkglieder des Werkzeugs (nicht gezeigt) des chirurgischen Instruments 52 bewegen bzw. verschwenken.

Figur 4 zeigt eine perspektivische Ansicht einer an einem Roboterarm (nicht gezeigt) anbringbaren Antriebseinheit 114. Die Antriebseinheit 114 weist eine Schnittstelle 112 zur Kopplung mit dem Lenkgetriebe 10 auf. An der Schnittstelle 112 sind vier Motortreibscheiben 140 angeordnet, die mit den Antriebsscheiben 40, 42, 44, 48 koppelbar sind, um die entsprechenden Antriebsspindeln 34, 36, 38 des Lenkgetriebes 10 anzutreiben. Die Antriebseinheit 102 und auch Teile der daran angeordneten Führungseinrichtung 142, über die die Antriebseinheit 114 an dem Roboterarm anbringbar ist, sind gemäß Figur 4 mit einem sterilen Überzug 144 überzogen. Der sterile Überzug 144 umschließt die Antriebseinheit 114, gibt dabei jedoch die Schnittstelle 112 frei. Dadurch kann die zweite Schnittstelle 50 des Lenkgetriebes 10 direkt mit der Schnittstelle 112 der Antriebseinheit 114 gekoppelt werden. Dies bedeutet, dass die unsterilen Antriebe mit ihren unsterilen Motortreibscheiben 140 nicht von dem sterilen Überzug 144 oder einem zusätzlichen Steriladapter abgedeckt werden müssen, da die unsterile Schnittstelle 112 der Antriebseinheit 114 durch das sterile Lenkgetriebe 10 vollständig abgedeckt wird.

Figur 5 zeigt eine perspektivische Ansicht der Antriebseinheit 114 im mit dem Lenkgetriebe 10 gekoppelten Zustand. Das Lenkgetriebe 10 ist steril. Das Lenkgetriebe 10 wird über seine zweite Schnittstelle 50 mit der Schnittstelle 112 der Antriebseinheit 114 gekoppelt. Diese Kopplung erfolgt in Richtung der Längsachse L des Lenkgetriebes 10, d.h. in Richtung der Drehachsen der Antriebsspindeln, die sich parallel zur Längsachse L erstrecken. Das sterile Lenkgetriebe 10 deckt somit die unsterilen Antriebe der Antriebseinheit 114 ab. Das Lenkgetriebe 10 kann während einer Operation durchgängig an der Antriebseinheit 114 verbleiben und die unsterilen Antriebe der Antriebseinheit 114 während der gesamten Operation abdecken.

Figur 6 zeigt eine perspektivische Ansicht des gesamten Antriebssystems 150. Das Antriebssystem 150 umfasst die Antriebseinheit 114, das Lenkgetriebe 10 und das chirurgische Instrument 52. Die Antriebseinheit 114, das Lenkgetriebe 10 und das chirurgischen Instrument 52 sind gemäß Figur 6 miteinander gekoppelt. Das chirurgische Instrument 52 wurde in Richtung S auf das Lenkgetriebe 10 aufgesetzt und über die Schnittstelle 14 des Lenkgetriebes 10 (siehe Figuren 1 bis 5) mit dem Lenkgetriebe 10 gekoppelt. Der Schaft 102 des chirurgischen Instruments 52 erstreckt sich durch eine Führungsöffnung 146 an einem Führungsvorsprung 148 der Führungseinrichtung 156.

Die vorliegende Erfindung betrifft ein Lenkgetriebe 10 für ein chirurgisches Instrument 52, wobei das Lenkgetriebe 10 ein Getriebegehäuse 12 und eine Schnittstelle 14 zur Kopplung mit einem chirurgischen Instrument 52 aufweist, wobei die Schnittstelle 14 derart ausgebildet ist, dass das chirurgische Instrument 52 in einer Richtung S senkrecht zur Längsachse L des Lenkgetriebes 10 mit dem Lenkgetriebe 10 koppelbar ist, wobei die Schnittstelle 14 umfasst: einen Gehäusebereich 16 des Getriebegehäuses 12, der zur Kopplung mit einem Gehäuse 54 des chirurgischen Instruments 52 ausgebildet ist, und eine Lenkklammer 18 zur Betätigung einer Lenkeinrichtung 54 des chirurgischen Instruments 52, wobei die Lenkklammer 18 mit einer Taumelscheibe 60 der Lenkeinrichtung 54 des chirurgischen Instruments 52 über die Schnittstelle 14 koppelbar ist.

### Bezugszeichenliste

- 10: Lenkgetriebe
- 12: Getriebegehäuse
- 14: Schnittstelle
- 16: Gehäusebereich
- 18: Lenkklammer
- 20: elastischer Abschnitt
- 22, 24: Schieber
- 26: Kugelkopf
- 28: Kugelpfannen
- 30: Zugklaue
- 32: Aufnahme
- 34, 36, 38: Antriebsspindeln
- 40, 42, 44: Antriebsscheiben
- 46: Stirnrad
- 48: Antriebsscheibe
- 50: Schnittstelle
- 52: chirurgisches Instrument
- 54: Gehäuse
- 56: Lenkeinrichtung
- 58: Schaftwelle
- 60: Taumelscheibe
- 62: Lenkdrähte
- 64: Öffnung
- 66: Umlaufende Nut
- 68, 70: Kontaktflächen
- 72: Befestigungselemente
- 74: Zughülse
- 76: Wälzlager
- 78: Zahnrad
- 80: Aufnahmebereich
- 82: Aussparung
- 84: Außenseite
- 86: Rand
- 88: Anlagefläche
- 90: Oberseite
- 92, 94: Klammerarme
- 96: Klammeröffnung
- 98, 100: Kontaktflächen
- 102: Schaft
- 104: Welle
- 106: Zahnradgetriebe
- 108, 110: Zahnräder
- 112: Schnittstelle
- 114: Antriebseinheit
- 116: Kopplungsfläche
- 118, 120: Wälzlager
- 122, 124: Lagerstellen
- 126: Befestigungsöffnungen
- 128: Welle
- 130: Zahnrad
- 132: Stirnrad
- 134: Zahnrad
- 136: Wandabschnitt
- 138: Verbindungsstück
- 140: Motortreibscheiben
- 142: Führungseinrichtung
- 144: Steriler Überzug
- 146: Führungsöffnung
- 148: Führungsvorsprung
- 150: Antriebssystem
- S: Richtung
- L: Längsachse

## Patentansprüche

1. Lenkgetriebe (10) für ein chirurgisches Instrument (52),
wobei das Lenkgetriebe (10) ein Getriebegehäuse (12) und eine Schnittstelle (14) zur Kopplung mit einem chirurgischen Instrument (52) aufweist,
wobei die Schnittstelle (14) derart ausgebildet ist, dass das chirurgische Instrument (52) in einer Richtung (S) senkrecht zur Längsachse (L) des Lenkgetriebes (10) mit dem Lenkgetriebe (10) koppelbar ist,
wobei die Schnittstelle (14) umfasst:
einen Gehäusebereich (16) des Getriebegehäuses (12), der zur Kopplung mit einem Gehäuse (54) des chirurgischen Instruments (52) ausgebildet ist, und
eine Lenkklammer (18) zur Betätigung einer Lenkeinrichtung (54) des chirurgischen Instruments (52), wobei die Lenkklammer (18) mit einer Taumelscheibe (60) der Lenkeinrichtung (54) des chirurgischen Instruments (52) über die Schnittstelle (14) koppelbar ist.

2. Lenkgetriebe (10) nach Anspruch 1,
wobei die Lenkklammer (18) derart ausgebildet ist, dass die Lenkklammer (18) in einer Richtung (S) senkrecht zur Längsachse (L) mit der Taumelscheibe (60) des chirurgischen Instruments (52) in Eingriff bringbar ist.

3. Lenkgetriebe (10) nach Anspruch 1 oder 2,
wobei die Lenkklammer (18) wenigstens einen elastischen Abschnitt (20) aufweist.

4. Lenkgetriebe (10) nach einem der Ansprüche 1 bis 3,
wobei das Lenkgetriebe (10) wenigstens zwei Schieber (22, 24) aufweist, die jeweils wenigstens einen Kugelkopf (26) aufweisen, wobei die Lenkklammer (18) Kugelpfannen (28) aufweist, mit denen sie an den Kugelköpfen (26) angebracht ist.

5. Lenkgetriebe (10) nach Anspruch 3 oder 4,
wobei der wenigstens eine elastische Abschnitt (20) der Lenkklammer (18) zwischen den Kugelpfannen (28) angeordnet ist.

6. Lenkgetriebe (10) nach einem der Ansprüche 1 bis 5,
wobei die Schnittstelle (14) eine Zugklaue (30) aufweist, die zur Kopplung mit der Lenkeinrichtung (56) des chirurgischen Instruments (52) ausgebildet ist, wobei die Zugklaue (30) wenigstens eine Aufnahme (32) aufweist, die derart ausgebildet ist, dass die Zugklaue (30) in einer Richtung (S) senkrecht zur Längsachse (L) des Lenkgetriebes (10) mit der Lenkeinrichtung (54) des chirurgischen Instruments (50) in Eingriff bringbar ist.

7. Lenkgetriebe (10) nach einem der Ansprüche 4 bis 6,
wobei das Lenkgetriebe (10) eine erste Antriebspindel (34) und eine zweite Antriebsspindel (36) aufweist, die jeweils einen der Schieber (22, 24) antreiben.

8. Lenkgetriebe (10) nach Anspruch 5 oder 6,
wobei die Zugklaue (30) über eine dritte Antriebsspindel (38) antreibbar ist.

9. Lenkgetriebe nach Anspruch 7 oder 8,
wobei sich die Antriebsspindeln (34, 36, 38) parallel zur Längsachse (L) des Lenkgetriebes (10) durch das Getriebegehäuse (12) erstrecken und an ihren Enden jeweils eine Antriebsscheibe (40, 42, 44) aufweisen, die mit Motortreibscheiben koppelbar sind.

10. Lenkgetriebe (10) nach einem der Ansprüche 1 bis 9,
wobei die Schnittstelle (14) wenigstens ein Stirnrad (46; 132) zum Antreiben eines Schafts (S) des chirurgischen Instruments (52) aufweist, wobei das Stirnrad (46) von einer weiteren Antriebsscheibe (48) antreibbar ist.

11. Lenkgetriebe (10) nach Anspruch 10,
wobei das wenigstens eine Stirnrad (46; 132) in Richtung der Längsachse (L) des Getriebegehäuses (12) zwischen der Lenkklammer (18) und der Zugklaue (30) oder an der der Zugklaue (30) abgewandten Seite der Lenkklammer (18) angeordnet ist.

12. Lenkgetriebe (10) einem der Ansprüche 9 bis 11,
wobei das Lenkgetriebe (10) eine weitere Schnittstelle (50) zur Verbindung mit einer Antriebseinheit (102) aufweist, wobei diese Schnittstelle (50) die Antriebsscheiben (40, 42, 44, 48) umfasst.

13. Chirurgisches Instrument (52) zur Kopplung mit einer Schnittstelle (14) eines Lenkgetriebes (10) nach einem der Ansprüche 1 bis 12,
wobei das chirurgische Instrument (52) ein Gehäuse (54) und eine in dem Gehäuse (54) angeordnete Lenkeinrichtung (56) aufweist, wobei die Lenkeinrichtung (56) zumindest eine Schaftwelle (58), eine Taumelscheibe (60) und mit der Taumelscheibe (60) verbundene Lenkdrähte (62) aufweist, wobei das Gehäuse (54) eine Öffnung (64) aufweist, über die die Lenkeinrichtung (56) mit dem Lenkgetriebe (10) koppelbar ist.

14. Chirurgisches Instrument (52) nach Anspruch 13,
wobei die Taumelscheibe (60) eine umlaufende Nut (66) hat, die axiale Kontaktflächen (68, 70) für die Lenkklammer (18) aufweist.

15. Chirurgisches Instrument (52) nach Anspruch 13 oder 14,
wobei die Taumelscheibe (60) Befestigungselemente (72) zur Befestigung der Lenkdrähte (62) an der Taumelscheibe (60) aufweist.

16. Chirurgisches Instrument (52) nach einem der Ansprüche 13 bis 15,
wobei die Lenkeinrichtung (56) eine Zughülse (74) und ein daran angeordnetes Wälzlager (76) umfasst, wobei die Zughülse (74) und das daran angeordnete Wälzlager (76) zum Einsetzen in die Aufnahme (32) der Zugklaue (30) des Lenkgetriebes (10) ausgebildet sind.

17. Chirurgisches Instrument (52) nach einem der Ansprüche 13 bis 16,
wobei die Lenkeinrichtung (56) ein Zahnrad (78; 134) aufweist, das an der Schaftwelle (58) anordnet und mit dem Stirnrad (46; 132) des Lenkgetriebes (10) in Eingriff bringbar ist.

18. Steriles Antriebssystem (150) für ein chirurgisches Instrument (52) nach einem der Ansprüche 13 bis 17, wobei das Antriebssystem (150) ein Lenkgetriebe (10) nach einem der Ansprüche 1 bis 12 und eine an einem Roboterarm anbringbare Antriebseinheit (114) aufweist, wobei das Antriebssystem (150) umfasst:
wenigstens einen sterilen Überzug (144), der zumindest die Antriebseinheit (114) umgibt, wobei der sterile Überzug (144) eine Schnittstelle (112) der Antriebseinheit (114) freigibt,
wobei das sterile Lenkgetriebe (10) mit der Schnittstelle (112) an der Antriebseinheit (114) direkt koppelbar ist, wobei die Schnittstelle (112) durch das Lenkgetriebe (10) abdeckt ist, und
wobei das sterile chirurgische Instrument (52) mit der Schnittstelle (14) an dem Lenkgetriebe (10) austauschbar gekoppelt ist.

19. System (150) nach Anspruch 18,
wobei der Überzug (144) eine Folie ist.

20. Verfahren zur Montage eines sterilen Antriebssystems (150) für ein chirurgisches Instrument (52) nach einem Ansprüche 13 bis 17, wobei das Antriebssystem (150) ein steriles Lenkgetriebe (10) nach einem der Ansprüche 1 bis 12 und eine an einem Roboterarm anbringbare Antriebseinheit (114) aufweist, wobei das Verfahren die folgenden Schritte umfasst:
Anbringen eines sterilen Überzugs (144) zumindest an der Antriebseinheit (114), wobei der sterile Überzug (144) eine Schnittstelle (112) der Antriebseinheit (114) freigibt,
Anbringen des sterilen Lenkgetriebes (10) an der Schnittstelle (112) an der Antriebseinheit (114), wobei das sterile Lenkgetriebe (10) direkt mit der Antriebseinheit (114) gekoppelt wird, und wobei das sterile Lenkgetriebe (10) die Schnittstelle (112) der Antriebseinheit (144) abdeckt, und
Koppeln eines sterilen chirurgischen Instruments (52) mit der Schnittstelle (14) an dem Lenkgetriebe (10), wobei das chirurgische Instrument (52) austauschbar ist.

## Claims

1. A steering mechanism (10) for a surgical instrument (52),
wherein the steering mechanism (10) has a mechanism housing (12) and an interface (14) for coupling to a surgical instrument (52),
wherein the interface (14) is designed in such manner that the surgical instrument (52) can be coupled to the steering mechanism (10) in a direction (S) perpendicular to the longitudinal axis (L) of the steering mechanism (10),
wherein the interface (14) comprises:
a housing region (16) of the mechanism housing (12), which is designed for coupling to a housing (54) of the surgical instrument (52), and
a steering clamp (18) for actuating a steering device (54) of the surgical instrument (52), wherein the steering clamp (18) can be coupled to a swash plate (60) of the steering device (54) of the surgical instrument (52) via the interface (14).

2. The steering mechanism (10) according to claim 1,
wherein the steering clamp (18) is designed in such manner that the steering clamp (18) can be brought into engagement with the swash plate (60) of the surgical instrument (52) in a direction (S) perpendicular to the longitudinal axis (L).

3. The steering mechanism (10) according to claim 1 or 2,
wherein the steering clamp (18) has at least one elastic section (20).

4. The steering mechanism (10) according to one of claims 1 to 3,
wherein the steering mechanism (10) has at least two sliders (22, 24), each of which has at least one ball head (26), wherein the steering clamp (18) has ball sockets (28) by means of which it is attached to the ball heads (26).

5. The steering mechanism (10) according to claim 3 or 4,
wherein the at least one elastic section (20) of the steering clamp (18) is arranged between the ball sockets (28).

6. The steering mechanism (10) according to one of claims 1 to 5,
wherein the interface (14) has a pull claw (30) which is designed for coupling to the steering device (56) of the surgical instrument (52), wherein the pull claw (30) has at least one receptacle (32) which is designed in such manner that the pull claw (30) can be brought into engagement with the steering device (54) of the surgical instrument (50) in a direction (S) perpendicular to the longitudinal axis (L) of the steering mechanism (10).

7. The steering mechanism (10) according to one of claims 4 to 6,
wherein the steering mechanism (10) has a first drive spindle (34) and a second drive spindle (36), each of which drives one of the sliders (22, 24).

8. The steering mechanism (10) according to claim 5 or 6,
wherein the pull claw (30) can be driven via a third drive spindle (38).

9. The steering mechanism according to claim 7 or 8,
wherein the drive spindles (34, 36, 38) extend through the mechanism housing (12) parallel to the longitudinal axis (L) of the steering mechanism (10) and each have, at their ends, a drive pulley (40, 42, 44) which can be coupled to motor drive pulleys.

10. The steering mechanism (10) according to one of claims 1 to 9,
wherein the interface (14) has at least one spur gear (46; 132) for driving a shaft (S) of the surgical instrument (52), wherein the spur gear (46) can be driven by a further drive pulley (48).

11. The steering mechanism (10) according to claim 10,
wherein the at least one spur gear (46; 132) is arranged in the direction of the longitudinal axis (L) of the mechanism housing (12) between the steering clamp (18) and the pull claw (30) or on the side of the steering clamp (18) facing away from the pull claw (30).

12. The steering mechanism (10) according to one of claims 9 to 11,
wherein the steering mechanism (10) has a further interface (50) for connection to a drive unit (102), wherein this interface (50) comprises the drive pulleys (40, 42, 44, 48).

13. A surgical instrument (52) for coupling to an interface (14) of a steering mechanism (10) according to one of claims 1 to 12,
wherein the surgical instrument (52) has a housing (54) and a steering device (56) arranged in the housing (54), wherein the steering device (56) has at least one drive shaft (58), a swash plate (60) and steering wires (62) connected to the swash plate (60), wherein the housing (54) has an opening (64) via which the steering device (56) can be coupled to the steering mechanism (10).

14. The surgical instrument (52) according to claim 13,
wherein the swash plate (60) has a circumferential groove (66) which has axial contact surfaces (68, 70) for the steering clamp (18).

15. The surgical instrument (52) according to claim 13 or 14,
wherein the swash plate (60) has fastening elements (72) for fastening the steering wires (62) to the swash plate (60).

16. The surgical instrument (52) according to one of claims 13 to 15,
wherein the steering device (56) comprises a pull sleeve (74) and a rolling bearing (76) arranged thereon, wherein the pull sleeve (74) and the rolling bearing (76) arranged thereon are designed for insertion into the receptacle (32) of the pull claw (30) of the steering mechanism (10).

17. The surgical instrument (52) according to one of claims 13 to 16, wherein the steering device (56) has a gear (78; 134) which is arranged on the drive shaft (58) and can be brought into engagement with the spur gear (46; 132) of the steering mechanism (10).

18. A sterile drive system (150) for a surgical instrument (52) according to one of claims 13 to 17, wherein the drive system (150) has a steering mechanism (10) according to one of claims 1 to 12 and a drive unit (114) which can be attached to a robotic arm, wherein the drive system (150) comprises: at least one sterile cover (144) that surrounds at least the drive unit (114), wherein the sterile cover (144) exposes an interface (112) of the drive unit (114),
wherein the sterile steering mechanism (10) can be directly coupled to the interface (112) on the drive unit (114), wherein the interface (112) is covered by the steering mechanism (10), and
wherein the sterile surgical instrument (52) is interchangeably coupled to the interface (14) on the steering mechanism (10).

19. The system (150) according to claim 18,
wherein the cover (144) is a film.

20. A method for assembling a sterile drive system (150) for a surgical instrument (52) according to one of claims 13 to 17, wherein the drive system (150) has a sterile steering mechanism (10) according to one of claims 1 to 12 and a drive unit (114) which can be attached to a robotic arm, wherein the method comprises the following steps:
attaching a sterile cover (144) at least to the drive unit (114), wherein the sterile cover (144) exposes an interface (112) of the drive unit (114),
attaching the sterile steering mechanism (10) to the interface (112) on the drive unit (114), wherein the sterile steering mechanism (10) is coupled directly to the drive unit (114), and wherein the sterile steering mechanism (10) covers the interface (112) of the drive unit (144), and
coupling a sterile surgical instrument (52) to the interface (14) on the steering mechanism (10), wherein the surgical instrument (52) is interchangeable.

## Revendications

1. Mécanisme de direction (10) destiné à un instrument chirurgical (52), dans lequel le mécanisme de direction (10) comporte un boîtier de mécanisme (12) et une interface (14) permettant l'accouplement à un instrument chirurgical (52),
dans lequel l'interface (14) est conçue de sorte que l'instrument chirurgical (52) peut être accouplé au mécanisme de direction (10) dans une direction (S) perpendiculaire à l'axe longitudinal (L) du mécanisme de direction (10),
dans lequel l'interface (14) comprend :
une zone de boîtier (16) du boîtier de mécanisme (12) conçue pour être accouplée à un boîtier (54) de l'instrument chirurgical (52), et
un étrier de direction (18) destiné à actionner un dispositif de direction (54) de l'instrument chirurgical (52), dans lequel l'étrier de direction (18) peut être accouplé à un plateau cyclique (60) du dispositif de direction (54) de l'instrument chirurgical (52) par l'intermédiaire de l'interface (14).

2. Mécanisme de direction (10) selon la revendication 1,
dans lequel l'étrier de direction (18) est conçu de sorte que l'étrier de direction (18) peut être mis en prise avec le plateau cyclique (60) de l'instrument chirurgical (52) dans une direction (S) perpendiculaire à l'axe longitudinal (L).

3. Mécanisme de direction (10) selon la revendication 1 ou 2,
dans lequel l'étrier de direction (18) comporte au moins une partie élastique (20).

4. Mécanisme de direction (10) selon l'une des revendications 1 à 3,
dans lequel le mécanisme de direction (10) comporte au moins deux coulisseaux (22, 24) qui comportent chacun au moins une tête sphérique (26), dans lequel l'étrier de direction (18) comporte des coussinets sphériques (28) avec lesquels il est fixé aux têtes sphériques (26).

5. Mécanisme de direction (10) selon la revendication 3 ou 4,
dans lequel au moins une partie élastique (20) de l'étrier de direction (18) est disposée entre les coussinets sphériques (28).

6. Mécanisme de direction (10) selon l'une des revendications 1 à 5,
dans lequel l'interface (14) comporte une griffe de traction (30) qui est conçue pour être accouplée au dispositif de direction (56) de l'instrument chirurgical (52), dans lequel la griffe de traction (30) comporte au moins un logement (32) qui est conçu de sorte que la griffe de traction (30) peut être mise en prise avec le dispositif de direction (54) de l'instrument chirurgical (50) dans une direction (S) perpendiculaire à l'axe longitudinal (L) du mécanisme de direction (10).

7. Mécanisme de direction (10) selon l'une des revendications 4 à 6,
dans lequel le mécanisme de direction (10) comporte une première broche d'entraînement (34) et une deuxième broche d'entraînement (36), qui entraînent chacune l'un des coulisseaux (22, 24).

8. Mécanisme de direction (10) selon la revendication 5 ou 6,
dans lequel la griffe de traction (30) peut être entraînée par une troisième broche d'entraînement (38).

9. Mécanisme de direction selon la revendication 7 ou 8,
dans lequel les broches d'entraînement (34, 36, 38) s'étendent parallèlement à l'axe longitudinal (L) du mécanisme de direction (10) à travers le boîtier de mécanisme (12) et comportent chacune à leurs extrémités une poulie d'entraînement (40, 42, 44) qui peut être accouplée à des poulies motrices de moteur.

10. Mécanisme de direction (10) selon l'une des revendications 1 à 9,
dans lequel l'interface (14) comporte au moins une roue dentée cylindrique (46 ; 132) permettant l'entraînement d'une tige (S) de l'instrument chirurgical (52), dans lequel la roue dentée cylindrique (46) peut être entraînée par une autre poulie d'entraînement (48).

11. Mécanisme de direction (10) selon la revendication 10,
dans lequel au moins une roue dentée cylindrique (46 ; 132) est disposée dans la direction de l'axe longitudinal (L) du boîtier de mécanisme (12) entre l'étrier de direction (18) et la griffe de traction (30) ou sur le côté de l'étrier de direction (18) opposé à la griffe de traction (30).

12. Mécanisme de direction (10) selon l'une des revendications 9 à 11,
dans lequel le mécanisme de direction (10) comporte une autre interface (50) permettant le raccordement à une unité d'entraînement (102), dans lequel cette interface (50) comprend les poulies d'entraînement (40, 42, 44, 48).

13. Instrument chirurgical (52) permettant l'accouplement à une interface (14) d'un mécanisme de direction (10) selon l'une des revendications 1 à 12,
dans lequel l'instrument chirurgical (52) comporte un boîtier (54) et un dispositif de direction (56) disposé dans le boîtier (54), dans lequel le dispositif de direction (56) comporte au moins un arbre de tige (58), un plateau cyclique (60) et des fils de direction (62) raccordés au plateau cyclique (60), dans lequel le boîtier (54) comporte une ouverture (64) par laquelle le dispositif de direction (56) peut être accouplé au mécanisme de direction (10).

14. Instrument chirurgical (52) selon la revendication 13,
dans lequel le plateau cyclique (60) présente une rainure circonférentielle (66) qui comporte des surfaces de contact axiales (68, 70) pour l'étrier de direction (18).

15. Instrument chirurgical (52) selon la revendication 13 ou 14,
dans lequel le plateau cyclique (60) comporte des éléments de fixation (72) permettant la fixation des fils de direction (62) au plateau cyclique (60).

16. Instrument chirurgical (52) selon l'une des revendications 13 à 15,
dans lequel le dispositif de direction (56) comprend une douille de traction (74) et un palier à roulement (76) disposé sur celui-ci, dans lequel la douille de traction (74) et le palier à roulement (76) disposé sur celui-ci sont conçus pour être insérés dans le logement (32) de la griffe de traction (30) du mécanisme de direction (10).

17. Instrument chirurgical (52) selon l'une des revendications 13 à 16, dans lequel le dispositif de direction (56) comporte une roue dentée (78 ; 134) qui est disposée sur l'arbre de tige (58) et qui peut être mise en prise avec la roue dentée cylindrique (46 ; 132) du mécanisme de direction (10).

18. Système d'entraînement stérile (150) destiné à un instrument chirurgical (52) selon l'une des revendications 13 à 17, dans lequel le système d'entraînement (150) comporte un mécanisme de direction (10) selon l'une des revendications 1 à 12 et une unité d'entraînement (114) pouvant être fixée à un bras de robot, dans lequel le système d'entraînement (150) comprend : au moins un revêtement stérile (144) qui entoure au moins l'unité d'entraînement (114), dans lequel le revêtement stérile (144) laisse libre une interface (112) de l'unité d'entraînement (114),
dans lequel le mécanisme de direction stérile (10) peut être accouplé directement à l'interface (112) de l'unité d'entraînement (114), dans lequel l'interface (112) est recouverte par le mécanisme de direction (10), et
dans lequel l'instrument chirurgical stérile (52) est accouplé de manière interchangeable à l'interface (14) sur le mécanisme de direction (10).

19. Système (150) selon la revendication 18,
dans lequel le revêtement (144) est un film.

20. Procédé d'assemblage d'un système d'entraînement stérile (150) destiné à un instrument chirurgical (52) selon l'une des revendications 13 à 17, dans lequel le système d'entraînement (150) comporte un mécanisme de direction stérile (10) selon l'une des revendications 1 à 12 et une unité d'entraînement (114) pouvant être fixée à un bras de robot, dans lequel le procédé comprend les étapes suivantes :
l'application d'un revêtement stérile (144) au moins sur l'unité d'entraînement (114), dans lequel le revêtement stérile (144) laisse libre une interface (112) de l'unité d'entraînement (114),
la fixation du mécanisme de direction stérile (10) à l'interface (112) de l'unité d'entraînement (114), dans lequel le mécanisme de direction stérile (10) est directement accouplé à l'unité d'entraînement (114) et dans lequel le mécanisme de direction stérile (10) recouvre l'interface (112) de l'unité d'entraînement (144), et
l'accouplement d'un instrument chirurgical stérile (52) à l'interface (14) sur le mécanisme de direction (10), dans lequel l'instrument chirurgical (52) est interchangeable.
